(19) **Europäisches Patentamt**
**European Patent Office**
**Office européen des brevets**

(11) **EP 4 094 771 A1**

(12) # EUROPEAN PATENT APPLICATION
published in accordance with Art. 153(4) EPC

(43) Date of publication:
**30.11.2022 Bulletin 2022/48**

(21) Application number: **21744730.9**

(22) Date of filing: **25.01.2021**

(51) International Patent Classification (IPC):
*A61K 36/53* (2006.01)   *A61K 36/896* (2006.01)
*A61K 36/489* (2006.01)   *A61K 36/75* (2006.01)
*A61K 36/756* (2006.01)   *A61P 17/00* (2006.01)
*A61P 39/06* (2006.01)   *A61P 29/00* (2006.01)

(52) Cooperative Patent Classification (CPC):
**A61K 36/489; A61K 36/53; A61K 36/75;
A61K 36/756; A61K 36/896; A61P 17/00;
A61P 29/00; A61P 39/06**

(86) International application number:
**PCT/KR2021/000986**

(87) International publication number:
**WO 2021/150088 (29.07.2021 Gazette 2021/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.01.2020 KR 20200009461**

(71) Applicant: **Lk. Inc.**
**Hwaseong-si, Gyeonggi-do 18469 (KR)**

(72) Inventors:
• **LEE, Jong Ryul**
  **Hwaseong-si, Gyeonggi-do 18479 (KR)**
• **SIN, Min Sub**
  **Seoul 05354 (KR)**
• **YUN, Young Min**
  **Seoul 02434 (KR)**

(74) Representative: **Schnappauf, Georg**
**ZSP Patentanwälte PartG mbB**
**Hansastraße 32**
**80686 München (DE)**

(54) **PHARMACEUTICAL COMPOSITION, PHARMACEUTICAL COMPOSITION FOR ANIMAL, AND FOOD COMPOSITION, EACH COMPRISING NATURAL SUBSTANCE EXTRACT, AND PREPARATION METHOD THEREFOR**

(57)    The present invention relates to a pharmaceutical composition, a pharmaceutical composition for animals, and a food composition, each composition comprising a Scutellaria baicalensis extract, a Liriope platyphylla extract, a Sophora flavescens extract, a Dictamnus root bark extract, and a Phellodendron amurense Ruprecht extract, and preparation methods therefor.

[FIG. 1]

**Description**

**Technical Field**

The present invention relates to a pharmaceutical composition, a pharmaceutical composition for animals, and a food composition, each composition comprising a Scutellaria baicalensis extract, a Liriope platyphylla extract, a Sophora flavescens extract, a Dictamnus root bark extract, and a Phellodendron amurense Ruprecht extract, and preparation methods therefor.

**Background Art**

Scutellaria baicalensis is a perennial herb which is in the class Dicotyledoneae, order Lamiales, family Lamiaceae and is distributed in Korea, China, Mongolia, and eastern Siberia. In traditional Oriental medicine, the root of Scutellaria baicalensis was used as an antipyretic, diuretic, antidiarrheal, or choleretic.

Liriope platyphylla (Broadleaf Liriope) is a perennial herb which is in the class Monocotyledoneae, order Liliales, family Liliaceae and is widely distributed around the world as well as in Northeast Asia such as Korea, Japan, China, and Taiwan. It has a superior ovary and its fruit is a round capsule. The fruit skin is peeled off early to expose the seeds which are purple-black. In traditional Oriental medicine, its tuber roots are used as a medicine such as tonic, antitussive, expectorant, and cardiotonic.

Sophora flavescens is a perennial herb which is in the class Dicotyledoneae, order Rosales, family Leguminosae and is also called thief s stick, bitter ginseng, or snake's shade tree. In traditional Oriental medicine, Sophora flavescens refers to its dried roots that are bitter in taste and have efficacy of ginseng, and is known to be prescribed for indigestion, neuralgia, hepatitis, jaundice, hemorrhoid, or the like. Also, in folk remedies, the stems or leaves of Sophora flavescens were decocted and used as an insecticide.

Dictamnus root bark refers to a root bark of Dictamnus dasycarpus Turcz., and was previously used not only for skin rash, skin erosion, eczema, rubella, or itching, which is caused by damp-heat, jaundice due to acute hepatitis, and damp-heat type paraplegia, but also cough, dry throat, or polydipsia. It is known to have an antipyretic action and a skin fungal inhibitory action as pharmacological actions.

Phellodendron bark refers to a dried bark of Phellodendron amurense Ruprecht that belongs to family Rutaceae. It is obtained by peeling the bark from the tree trunk around the summer solstice, removing the coarse skin or having it chopped, and then performing drying in the sun. The Phellodendron bark thus obtained is used. The Phellodendron bark is known to lower blood sugar, inhibit growth of pneumococcus, Mycobacterium tuberculosis var. hominis, staphylococcus, and the like, inhibit proliferation of tumor cells, and have a bactericidal action. It is also known to promote secretion of gastric juice when taken and to cause an increase in appetite.

Recently, regarding a number of inflammation or oxidation-related symptoms or diseases, there is an increasing demand for drugs for prevention or treatment thereof and food compositions for amelioration thereof, the drugs and the food compositions being derived from natural products.

**Disclosure of Invention**

**Technical Problem**

An object of the present invention is, for example, to provide a pharmaceutical composition, a pharmaceutical composition for animals, and a food composition, each composition comprising a Scutellaria baicalensis extract, a Liriope platyphylla extract, a Sophora flavescens extract, a Dictamnus root bark extract, and a Phellodendron bark extract, and preparation methods therefor.

**Solution to Problem**

The present inventors have found that use of at least one natural material selected from the group consisting of Scutellaria baicalensis, Liriope platyphylla, Sophora flavescens, Dictamnus root bark, and Phellodendron bark, which are traditionally known as medicinal herbs, makes it possible to prepare a pharmaceutical composition having a preventive or therapeutic effect on skin-related diseases in humans or animals, or a food composition having an ameliorating effect on the same diseases, and thus have completed the present invention. Hereinafter, the present invention will be described in more detail.

The present invention relates to a pharmaceutical composition for preventing or treating a skin-related disease in humans or animals, the pharmaceutical composition comprising at least one extract selected from the group consisting of a Scutellaria baicalensis extract, a Liriope platyphylla extract, a Sophora flavescens extract, a Dictamnus root bark extract, and a Phellodendron bark extract.

In an embodiment, the composition may comprise all of the Scutellaria baicalensis extract, the Liriope platyphylla extract, the Sophora flavescens extract, the Dictamnus root bark extract, and the Phellodendron bark extract.

In an embodiment, the Liriope platyphylla extract may be included in an amount of 50 to 150 parts by weight, specifically 80 to 120 parts by weight, more specifically 90 to 110 parts by weight, for example, 100 parts by weight, based on 100 parts by weight of the Scutellaria baicalensis extract.

The Sophora flavescens extract may be included in an amount of 50 to 150 parts by weight, specifically 80 to 120 parts by weight, more specifically 90 to 110 parts by weight, for example, 100 parts by weight, based on 100 parts by weight of the Scutellaria baicalensis extract.

The Dictamnus root bark extract may be included in an amount of 50 to 150 parts by weight, specifically 80 to 120 parts by weight, more specifically 90 to 110 parts by weight, for example, 100 parts by weight, based on 100 parts by weight of the Scutellaria baicalensis extract.

The Phellodendron bark extract may be included in an amount of 50 to 150 parts by weight, specifically 80 to 120 parts by weight, more specifically 90 to 110 parts by weight or 95 to 105 parts by weight, for example, 100 parts by weight, based on 100 parts by weight of the Scutellaria baicalensis extract.

Specifically, the pharmaceutical composition may comprise 50 to 150 parts by weight of the Liriope platyphylla extract, 150 to 250 parts by weight of the Sophora flavescens extract, 50 to 150 parts by weight of the Dictamnus root bark extract, and 50 to 150 parts by weight of the Phellodendron bark extract, based on 100 parts by weight of the Scutellaria baicalensis extract.

In an embodiment, a content of the extract(s) in the composition may be 0.5 to 5 v/v%, specifically 1 to 4 v/v%, more specifically 1.5 to 2.5 v/v%, for example, 2 v/v% or 3 v/v%.

For example, each extract in the pharmaceutical composition may be an extract extracted using one or more solvents selected from the group consisting of water, an organic solvent, and a mixture thereof. Specifically, the organic solvent may be, for example, alcohol, and more specifically, butylene glycol or ethanol.

The extract may be, for example, a liquid material obtained by performing maceration, heating, or filtration at room temperature, or may be obtained by evaporating and drying a solvent in the liquid material.

For the drying, any known drying method may be used without limitation. Specifically, the drying may be carried out by a spray drying method or a freeze drying method. More specifically, the drying may be carried out by a spray drying method.

The Scutellaria baicalensis extract, the Liriope platyphylla extract, the Sophora flavescens extract, the Dictamnus root bark extract, or the Phellodendron bark extract may be one extracted at a temperature of 40 to 55°C, specifically 47 to 52°C, for example, 50°C.

In addition, the Scutellaria baicalensis extract, the Liriope platyphylla extract, the Sophora flavescens extract, the Dictamnus root bark extract, or the Phellodendron bark extract may be one extracted for 60 to 80 hours, specifically 68 to 75 hours, for example, 72 hours.

For example, the extract may be one extracted at a temperature of 47 to 52°C for 68 to 75 hours.

In a case where the respective extracts are included in weight ratios or contents in the composition as mentioned above, or extracted using the conditions for extraction solvent, extraction temperature, and extraction time as mentioned above, or obtained by drying a solvent according to the drying method as mentioned above, each of the extracts may have an excellent antioxidant effect due to its high ability to inhibit reactive oxygen species, have an excellent anti-inflammatory effect due to its high ability to inhibit NO synthesis and its high ability to inhibit expression of inflammatory cytokines, and have an excellent skin whitening effect due to its high ability to inhibit production of melanin, which allows a more excellent preventive or therapeutic effect on a skin-related disease, for example, atopic dermatitis.

The term "prevention" may refer to any action that inhibits or delays onset of a skin-related disease in an individual by administration of the pharmaceutical composition according to an embodiment.

The term "treatment" may refer to any action that ameliorates or beneficially alters symptoms of a skin-related disease in an individual by administration of the pharmaceutical composition according to an embodiment.

In an embodiment, the composition may exhibit an antioxidant effect by inhibiting reactive active oxygen species, exhibit an anti-inflammatory effect by inhibiting NO synthesis and inhibiting expression of inflammatory cytokines, and exhibit a skin whitening effect by inhibiting production of melanin.

In addition, the antioxidant effect, the anti-inflammatory effect, and the skin whitening effect allow the composition to exhibit a preventive or therapeutic effect on a skin-related disease.

In an embodiment, the skin-related disease may be at least one disease selected from the group consisting of skin wrinkles, photoaging, melanosis, skin pigmentation, skin burns, skin inflammation, skin cancer, melasma, freckles, lentigines, nevus, psoriasis, atopic dermatitis, alopecia areata, vitiligo, athlete's foot, and dandruff. Specifically, the skin-related disease may be at least one disease selected from the group consisting of skin pigmentation, skin inflammation, psoriasis, and atopic dermatitis. More specifically, the skin-related disease may be atopic dermatitis.

The atopic dermatitis is one of representative allergic diseases along with asthma, allergic rhinitis, and chronic urticaria, and is an eczematous skin disease that chronically recurs and is accompanied by severe itching.

It is known that patients with atopic dermatitis are more damaged by reactive oxygen species (ROS) due to a decrease in malondialdehyde and antioxidants. Thus, it is known that antioxidants may be effective in treating atopic dermatitis (Journal of Clinical and Diagnostic Research, N. Sivaranjani etc. 2013; 7(12): 2683-2685).

In addition, the pharmaceutical composition may be used as a composition for blocking blue light, or preventing or treating tinea cruris or tinea, and may have antioxidant, anti-inflammatory, antifungal, antiviral, antibacterial, cell-regenerating, and whitening effects.

Specifically, the pharmaceutical composition may have an antioxidant effect. Specifically, the pharmaceutical composition may have an ability to inhibit reactive oxygen species. For example, based on 2 v/v% of the extract, the pharmaceutical composition may have a rate of conversion of DCFH to DCF by reactive oxygen species which is 41 to 80% or 41 to 70%, specifically 43 to 50% or 51 to 70%, and more specifically 44 to 48% or 64 to 68%, as compared with an untreated control.

In addition, the pharmaceutical composition may have an anti-inflammatory effect, and specific examples of the anti-inflammatory effect include inhibiting NO synthesis or inhibiting expression of inflammatory cytokines. For example, based on 2 v/v% of the extract that is an active ingredient, the pharmaceutical composition may have a NO synthesis inhibition ability of 65 to 99.9%, specifically 67 to 75% or 95 to 99%, as calculated by Expression 1.

[Expression 1]

$$\text{NO synthesis inhibition ability (\%)} = \{1 - (\text{Slope of NO synthesis amount when sample is added})\}/(\text{NO synthesis amount when no sample is added}) \times 100$$

In addition, the composition may have an effect of inhibiting production of melanin. For example, based on 2 v/v% of the extract that is an active ingredient, the composition may have an intracellular melanin production inhibition rate of 5 to 30%, specifically 10 to 20%, for example, 12 to 16%, as calculated by Expression 2.

[Expression 2]

$$\text{Melanin production inhibition rate (\%)} = \{(\text{Melanin amount when no sample is added}) - (\text{Melanin amount when sample is added})\}/(\text{Melanin amount when no sample is added}) \times 100$$

In addition, the composition may have an anti-atopic dermatitis effect. For example, in a case where the composition is orally administered, it is possible to identify that atopic dermatitis is treated through measurement of ear thickness, measurement of lymph node weight, identification of clinical symptoms, skin irritation index, and the like.

The pharmaceutical composition may comprise an active ingredient alone or together with one or more pharmaceutically acceptable carriers, excipients, or diluents.

Specifically, the carrier may be, for example, a colloidal suspension, powder, saline, lipid, liposome, microspheres, or nano-spherical particles. The active ingredient may form a complex with a vehicle or may be associated therewith, and may be delivered *in vivo* using a vehicle system known in the art, such as lipids, liposomes, microparticles, gold, nanoparticles, polymers, condensation agents, polysaccharides, polyamino acids, dendrimers, saponins, adsorption enhancers, or fatty acids.

In a case where the pharmaceutical composition is made into a formulation, the formulation may be prepared using commonly used diluents or excipients such as lubricants, sweeteners, flavoring agents, emulsifiers, suspending agents, preservatives, fillers, extenders, binders, wetting agents, disintegrants, and surfactants. Solid formulations for oral administration may include tablets, pills, powders, granules, capsules, and the like, and these solid preparations may be made by mixing the composition with at least one excipient (such as starch, calcium carbonate, sucrose or lactose, gelatin). In addition, besides simple excipients, lubricants such as magnesium stearate and talc may also be used. Liquid formulations for oral administration may include suspensions, solutions, emulsions, syrups, and the like. These liquid formulations may comprise various excipients such as wetting agents, sweetening agents, fragrances, and preservatives, in addition to water and liquid paraffin which are commonly used simple diluents. Formulations for parenteral administration may include sterile aqueous solutions, non-aqueous solutions, suspensions, emulsions, freeze-dried preparations, and suppositories. For the non-aqueous solutions and suspensions, propylene glycol, polyethylene glycol, vegetable oil such as olive oil, injectable ester such as ethyl oleate, or the like may be used. As a base of the suppository, Witepsol, macrogol, Tween 61, cacao butter, laurin fat, glycerogelatin, or the like may be used. In a case where the pharmaceutical composition is prepared in the form of eye drops, known diluents or excipients or the like may be used.

The term "pharmaceutically acceptable" refers to properties that are non-toxic to cells or humans in a case of being exposed to the composition.

The term "individual" refers to a subject in need of treatment for a skin-related disease. Specifically, the individual may refer to an animal. More specifically, the individual may refer to a human or non-human primate, or a mammal such as mice, dogs, cats, horses, and cattle.

The pharmaceutical composition may be administered orally or parenterally (for example, intramuscularly, intravenously, intraperitoneally, subcutaneously, intradermally, or topically) depending on its intended application.

The term "administration" means introducing a predetermined substance into a subject by an appropriate method.

In addition, the pharmaceutical composition is administered in a pharmaceutically effective amount. The term "pharmaceutically effective amount" means an amount sufficient to treat a disease with a reasonable benefit/risk ratio applicable to medical treatment, and an effective dose level may be determined depending on factors including the patient's disease type, severity, activity of drug, and sensitivity to drug, administration time, administration route and excretion rate, duration of treatment, and concurrently used drug(s), and other factors well known in the medical field. Specifically, the pharmaceutical composition may be administered at a concentration of 0.1 to 10 mg/mL, specifically 1 to 5 mg/mL, and more specifically 2 to 4 mg/mL (based on the volume), and may be administered at a dose of 100 to 1,000 mpk, specifically 300 to 800 mpk, and more specifically 400 to 600 mpk (based on the weight of an individual being administered). The administration may be made once a day or several times a day.

The pharmaceutical composition according to an embodiment may be administered individually or in combination with another therapeutic agent for a skin-related disease. The pharmaceutical composition may be administered simultaneously, separately, or sequentially with a known therapeutic agent for a skin-related disease. The pharmaceutical composition may be administered, and may be administered once or multiple times. Taking all of the above factors into consideration, it is important to administer a minimum amount that allows the maximum effect to be achieved without adverse effects, which can be easily determined by those skilled in the art.

In addition, the present invention relates to a method for preventing or treating a skin-related disease, comprising a step of administering the pharmaceutical composition to an individual in need thereof.

The "pharmaceutical composition", "individual", "administration", "prevention", "treatment", "skin-related disease", and the like may be within the above-described range.

In addition, the present invention relates to a preparation method of a pharmaceutical composition for the prevention or treatment of a skin-related disease in humans or animals, comprising a step of extracting at least one natural material selected from the group consisting of Scutellaria baicalensis, Liriope platyphylla, Sophora flavescens, Dictamnus root bark, and Phellodendron bark.

The "Scutellaria baicalensis", "Liriope platyphylla", "Sophora flavescens", "Dictamnus root bark", "Phellodendron bark", "skin-related disease", "pharmaceutical composition", "prevention", "treatment", and the like may be within the above-described range.

In an embodiment of the present invention, the natural material may include all of Scutellaria baicalensis, Liriope platyphylla, Sophora flavescens, Dictamnus root bark, and Phellodendron bark, provided that the extraction may be carried out for each of Scutellaria baicalensis, Liriope platyphylla, Sophora flavescens, Dictamnus root bark, and Phellodendron bark.

In addition, in an embodiment, the preparation method of a pharmaceutical composition may further comprise a step of mixing, based on 100 parts by weight of the Scutellaria baicalensis extract, 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, to obtain a mixture.

Specifically, the preparation method of a pharmaceutical composition may comprise steps of mixing Scutellaria baicalensis, Liriope platyphylla, Sophora flavescens, Dictamnus root bark, and Phellodendron bark, and extracting the mixture to prepare a mixture that comprises a Scutellaria baicalensis extract, a Liriope platyphylla extract, a Sophora flavescens extract, a Dictamnus root bark extract, and a Phellodendron bark extract.

In addition, in an embodiment, the preparation method of a pharmaceutical composition may further comprise drying the mixture.

For the drying, any known drying method may be used without limitation. Specifically, the drying may be carried out by a spray drying method or a freeze drying method. More specifically, the drying may be carried out by a spray drying method.

The extraction may be performed at a temperature of 40 to 55°C, specifically 47 to 52°C, for example, 50°C.

In addition, the extraction may be performed for 60 to 80 hours, specifically 68 to 75 hours, for example, 72 hours.

In addition, the extraction may be performed with at least one solvent selected from the group consisting of water, an organic solvent, and a mixture thereof.

For example, the extraction may be performed using a mixture of water and an organic solvent as an extraction solvent at a temperature of 47 to 52°C for 68 to 75 hours.

In a case where the extracts in the pharmaceutical composition are obtained using the conditions for temperature, time, and extraction solvent as mentioned above, or such extracts are dried according to the drying method as mentioned above, the pharmaceutical composition may have an excellent antioxidant effect due to its high ability to inhibit reactive oxygen species, have an excellent anti-inflammatory effect due to its high ability to inhibit NO synthesis and its high ability to inhibit expression of inflammatory cytokines, and have an excellent skin whitening effect due to its high ability to inhibit production of melanin, which allows a more excellent preventive or therapeutic effect on a skin-related disease, for example, atopic dermatitis.

In addition, the present invention relates to a food composition for preventing or ameliorating a skin-related disease, comprising at least one extract selected from the group consisting of a Scutellaria baicalensis extract, a Liriope platyphylla extract, a Sophora flavescens extract, a Dictamnus root bark extract, and a Phellodendron bark extract. The food composition may be a composition for health functional foods, functional foods, or food additives.

In an embodiment, the composition may comprise all of the Scutellaria baicalensis extract, the Liriope platyphylla extract, the Sophora flavescens extract, the Dictamnus root bark extract, and the Phellodendron bark extract.

In an embodiment, the Liriope platyphylla extract may be included in an amount of 50 to 150 parts by weight, specifically 80 to 120 parts by weight, more specifically 90 to 110 parts by weight, for example, 100 parts by weight, based on 100 parts by weight of the Scutellaria baicalensis extract.

The Sophora flavescens extract may be included in an amount of 50 to 150 parts by weight, specifically 80 to 120 parts by weight, more specifically 90 to 110 parts by weight, for example, 100 parts by weight, based on 100 parts by weight of the Scutellaria baicalensis extract.

The Dictamnus root bark extract may be included in an amount of 50 to 150 parts by weight, specifically 80 to 120 parts by weight, more specifically 90 to 110 parts by weight, for example, 100 parts by weight, based on 100 parts by weight of the Scutellaria baicalensis extract.

The Phellodendron bark extract may be included in an amount of 50 to 150 parts by weight, specifically 80 to 120 parts by weight, more specifically 90 to 110 parts by weight or 95 to 105 parts by weight, for example, 100 parts by weight, based on 100 parts by weight of the Scutellaria baicalensis extract.

Specifically, the food composition may comprise 50 to 150 parts by weight of the Liriope platyphylla extract, 150 to 250 parts by weight of the Sophora flavescens extract, 50 to 150 parts by weight of the Dictamnus root bark extract, and 50 to 150 parts by weight of the Phellodendron bark extract, based on 100 parts by weight of the Scutellaria baicalensis extract.

In an embodiment, a content of the extract(s) in the composition may be 0.5 to 5 v/v%, specifically 1 to 4 v/v%, more specifically 1.5 to 2.5 v/v%, for example, 2 v/v% or 3 v/v%.

For example, each extract in the food composition may be an extract extracted using one or more solvents selected from the group consisting of water, an organic solvent, and a mixture thereof. Specifically, the organic solvent may be, for example, alcohol, and more specifically, butylene glycol or ethanol.

The extract may be, for example, a liquid material obtained by performing maceration, heating, or filtration at room temperature, or may be obtained by evaporating and drying a solvent in the liquid material.

For the drying, any known drying method may be used without limitation. Specifically, the drying may be carried out by a spray drying method or a freeze drying method. More specifically, the drying may be carried out by a spray drying method.

The Scutellaria baicalensis extract, the Liriope platyphylla extract, the Sophora flavescens extract, the Dictamnus root bark extract, or the Phellodendron bark extract may be one extracted at a temperature of 40 to 55°C, specifically 47 to 52°C, for example, 50°C.

In addition, the Scutellaria baicalensis extract, the Liriope platyphylla extract, the Sophora flavescens extract, the Dictamnus root bark extract, or the Phellodendron bark extract may be one extracted for 60 to 80 hours, specifically 68 to 75 hours, for example, 72 hours.

For example, the extract may be one extracted at a temperature of 47 to 52°C for 68 to 75 hours.

In a case where the extracts are included in weight ratios or contents in the composition as mentioned above, or extracted using the conditions for extraction solvent, extraction temperature, and extraction time as mentioned above, or obtained by drying a solvent according to the drying method as mentioned above, each of the extracts may have an excellent antioxidant effect due to its high ability to inhibit reactive oxygen species, have an excellent anti-inflammatory effect due to its high ability to inhibit NO synthesis and its high ability to inhibit expression of inflammatory cytokines, and have an excellent skin whitening effect due to its high ability to inhibit production of melanin, which allows a more excellent preventive or ameliorating effect on a skin-related disease, for example, atopic dermatitis.

The term "prevention" may refer to any action that inhibits or delays onset of a skin-related disease in an individual by ingestion of the food composition according to an embodiment.

The term "amelioration" may refer to any action that at least decreases parameters associated with the condition being treated, for example, severity of symptoms. Here, for the prevention or amelioration of a skin-related disease, the food composition may be used separately from or simultaneously with a medicament for treatment of the disease before or after onset of the disease.

In an embodiment, the composition may exhibit an antioxidant effect by inhibiting reactive active oxygen species, exhibit an anti-inflammatory effect by inhibiting NO synthesis and inhibiting expression of inflammatory cytokines, and exhibit a skin whitening effect by inhibiting production of melanin.

In addition, the antioxidant effect, the anti-inflammatory effect, and the skin whitening effect allow the composition to exhibit a preventive or ameliorating effect on a skin-related disease.

In an embodiment, the skin-related disease may be at least one disease selected from the group consisting of skin wrinkles, photoaging, melanosis, skin pigmentation, skin burns, skin inflammation, skin cancer, melasma, freckles, lentigines, nevus, psoriasis, atopic dermatitis, alopecia areata, vitiligo, athlete's foot, and dandruff. Specifically, the skin-related disease may be at least one disease selected from the group consisting of skin pigmentation, skin inflammation, psoriasis, and atopic dermatitis. More specifically, the skin-related disease may be atopic dermatitis.

The atopic dermatitis is one of representative allergic diseases along with asthma, allergic rhinitis, and chronic urticaria, and is an eczematous skin disease that chronically recurs and is accompanied by severe itching.

It is known that patients with atopic dermatitis are more damaged by reactive oxygen species (ROS) due to a decrease in malondialdehyde and antioxidants. Thus, it is known that antioxidants may be effective in treating atopic dermatitis (Journal of Clinical and Diagnostic Research, N. Sivaranjani etc. 2013; 7(12): 2683-2685).

In addition, the food composition may be used as a composition for blocking blue light, or preventing or ameliorating tinea cruris or tinea, and may have antioxidant, anti-inflammatory, antifungal, antiviral, antibacterial, cell-regenerating, and whitening effects.

Specifically, the food composition may have an antioxidant effect. Specifically, the food composition may have an ability to inhibit reactive oxygen species. For example, based on 2 v/v% of the extract, the food composition may have a rate of conversion of DCFH to DCF by reactive oxygen species which is 41 to 80% or 41 to 70%, specifically 43 to 50% or 51 to 70%, and more specifically 44 to 48% or 64 to 68%, as compared with an untreated control.

In addition, the food composition may have an anti-inflammatory effect, and specific examples of the anti-inflammatory effect include inhibiting NO synthesis or inhibiting expression of inflammatory cytokines.

For example, based on 2 v/v% of the extract that is an active ingredient, the food composition may have a NO synthesis inhibition ability of 65 to 99.9%, specifically 67 to 75% or 95 to 99%, as calculated by Expression 1.

[Expression 1]

$$\text{NO synthesis inhibition ability (\%)} = \{1 - (\text{Slope of NO synthesis amount when sample is added})\}/(\text{NO synthesis amount when no sample is added}) \times 100$$

In addition, the composition may have an effect of inhibiting production of melanin. For example, based on 2 v/v% of the extract that is an active ingredient, the composition may have an intracellular melanin production inhibition rate of 5 to 30%, specifically 10 to 20%, for example, 12 to 16%, as calculated by Expression 2.

[Expression 2]

$$\text{Melanin production inhibition rate (\%)} = \{(\text{Melanin amount when no sample is added}) - (\text{Melanin amount when sample is added})\}/(\text{Melanin amount when no sample is added}) \times 100$$

In addition, specifically, the composition may have an anti-atopic dermatitis effect. For example, in a case where the composition is orally administered, it is possible to identify that atopic dermatitis is treated through measurement of ear thickness, measurement of lymph node weight, identification of clinical symptoms, skin irritation index, and the like.

In the food composition, the active ingredient may be added to food as it is or used together with another food or food ingredient, and may be appropriately used according to a conventional method. A mixing amount of the active ingredients may be appropriately determined depending on their intended use (for prevention or amelioration). In general, in the production of foods or beverages, the food composition may be added in an amount of specifically about 15% by weight or lower, and more specifically about 10% by weight or lower, based on the raw material. However, in a case where the food composition is ingested for a long period of time for the purpose of health and hygiene or for health control, the amount may be equal to or lower than the above-mentioned range.

The food composition may further comprise one or more of diluents, excipients, or additives such as carriers, fillers, extenders, binders, wetting agents, disintegrants, and surfactants, so that the food composition is formulated into one selected from the group consisting of tablets, pills, powders, granules, powders, capsules, and liquid formulations. Examples of the food to which the food composition is added include various foods, powders, granules, tablets, capsules, syrups, beverages, gums, tea, vitamin complexes, and health functional foods.

Specific examples of the carrier, excipient, diluent, and additive include at least one selected from the group consisting of lactose, dextrose, sucrose, sorbitol, mannitol, erythritol, starch, gum acacia, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methylhydroxybenzoate, propylhydroxybenzoate, talc, magnesium stearate, and mineral oil.

The food composition may contain, in addition to the active ingredient, other ingredients as essential ingredients without any particular limitation. For example, as in conventional beverages, various flavoring agents or natural carbohydrates may be contained as additional ingredients in the food composition. Examples of the above-mentioned natural carbohydrate include monosaccharides such as glucose and fructose; disaccharides such as maltose and sucrose; and polysaccharides, for example, conventional sugars such as dextrin and cyclodextrin, and sugar alcohols such as xylitol, sorbitol, and erythritol. For the flavoring agents, in addition to those mentioned above, natural flavoring agents (thaumatin, stevia extract (for example, rebaudioside A and glycyrrhizin)) and synthetic flavoring agents (for example, saccharin and aspartame) may be advantageously used. A proportion of the natural carbohydrate may be appropriately determined by selection of those skilled in the art.

In addition to those mentioned above, the food composition may contain various nutrients, vitamins, minerals (electrolytes), flavoring agents such as synthetic flavoring agents and natural flavoring agents, coloring agents and fillers (such as cheese and chocolate), pectic acid and its salts, alginic acid and its salts, organic acids, protective colloidal thickeners, pH adjusters, stabilizers, preservatives, glycerin, alcohols, carbonating agents used in carbonated beverages, and the like. These ingredients may be used individually or in combination, and proportions of these additives may also be appropriately selected by those skilled in the art.

In addition, in the present invention, the "functional food" or "health functional food" means food manufactured and processed using raw materials or ingredients which have useful functionality in the human body, and these foods include a food group, which is obtained by giving added value to foods using physical, biochemical, or bioengineering methods, or the like so that functions of the food in question act and manifest for a specific purpose, and foods, which are designed and processed so that regulatory functions of the food composition for controlling the biological defense rhythm, or preventing, recovering, or ameliorating a disease sufficiently manifest *in vivo*.

The term "individual" refers to a subject in need of prevention or amelioration of a skin-related disease. Specifically, the individual may refer to an animal. More specifically, the individual may refer to a human or non-human primate, or a mammal such as mice, dogs, cats, horses, and cattle.

In addition, the present invention relates to a method for preventing or ameliorating a skin-related disease, comprising a step of causing an individual in need thereof to ingest the food composition.

The "food composition", "individual", "prevention", "amelioration", "skin-related disease", and the like may be within the above-described range.

In addition, the present invention relates to a method for preparing a food composition for preventing or ameliorating a skin-related disease, comprising a step of extracting at least one natural material selected from the group consisting of Scutellaria baicalensis, Liriope platyphylla, Sophora flavescens, Dictamnus root bark, and Phellodendron bark.

The "Scutellaria baicalensis", "Liriope platyphylla", "Sophora flavescens", "Dictamnus root bark", "Phellodendron bark", "skin-related disease", "food composition", "prevention", "amelioration", and the like may be within the above-described range.

In an embodiment of the present invention, the natural material may include all of Scutellaria baicalensis, Liriope platyphylla, Sophora flavescens, Dictamnus root bark, and Phellodendron bark, provided that the extraction may be carried out for each of Scutellaria baicalensis, Liriope platyphylla, Sophora flavescens, Dictamnus root bark, and Phellodendron bark.

In addition, in an embodiment, the preparation method of a food composition may further comprise a step of mixing, based on 100 parts by weight of the Scutellaria baicalensis extract, 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, to obtain a mixture.

Specifically, the preparation method of a food composition may comprise steps of mixing Scutellaria baicalensis, Liriope platyphylla, Sophora flavescens, Dictamnus root bark, and Phellodendron bark, and extracting the mixture to prepare a mixture that comprises a Scutellaria baicalensis extract, a Liriope platyphylla extract, a Sophora flavescens extract, a Dictamnus root bark extract, and a Phellodendron bark extract.

In addition, in an embodiment, the preparation method of a food composition may further comprise drying the mixture.

For the drying, any known drying method may be used without limitation. Specifically, the drying may be carried out by a spray drying method or a freeze drying method. More specifically, the drying may be carried out by a spray drying method.

The extraction may be performed at a temperature of 40 to 55°C, specifically 47 to 52°C, for example, 50°C.

In addition, the extraction may be performed for 60 to 80 hours, specifically 68 to 75 hours, for example, 72 hours.

In addition, the extraction may be performed with at least one solvent selected from the group consisting of water, an organic solvent, and a mixture thereof.

For example, the extraction may be performed using a mixture of water and an organic solvent as an extraction solvent at a temperature of 47 to 52°C for 68 to 75 hours.

In a case where the extracts in the food composition are obtained using the conditions for temperature, time, and extraction solvent as mentioned above, or such extracts are dried according to the drying method as mentioned above, the food composition may have an excellent antioxidant effect due to its high ability to inhibit reactive oxygen species, have an excellent anti-inflammatory effect due to its high ability to inhibit NO synthesis and its high ability to inhibit expression of inflammatory cytokines, and have an excellent skin whitening effect due to its high ability to inhibit production of melanin, which allows a more excellent preventive or ameliorating effect on a skin-related disease, for example, atopic dermatitis.

Hereinafter, the present invention will be described in detail by way of examples to help understand the present invention. However, the embodiments according to the present invention may be modified into other forms, and the scope of the present invention should not be construed as being limited to the following examples.

The embodiments of the present invention are provided to more completely explain the present invention to those of ordinary skill in the art.

**Advantageous Effects of Invention**

The composition of the present invention has excellent antioxidant and anti-inflammatory effects, and thus can be usefully used as a composition for preventing or treating various diseases in a human or animal, or as a food composition having an ameliorating effect on such diseases.

**Brief Description of Drawings**

FIG. 1 illustrates results showing an ability to inhibit reactive oxygen species of the composition according to an embodiment of the present invention.

FIG. 2 illustrates results showing an ability to inhibit NO synthesis of the composition according to an embodiment of the present invention.

FIG. 3 illustrates results showing an intracellular melanin production inhibition rate of the composition according to an embodiment of the present invention.

FIG. 4 illustrates results obtained by evaluating cytotoxicity in human-derived keratinocytes (HaCaT) of the composition according to an embodiment of the present invention.

FIG. 5 illustrates results obtained by evaluating NO production (NO assay) of the composition according to an embodiment of the present invention.

FIG. 6 illustrates results obtained by evaluating inhibition of expression of inflammatory cytokines of 1 mg/mL of the composition according to an embodiment of the present invention.

FIG. 7 illustrates results obtained by evaluating inhibition of expression of inflammatory cytokines of 3 mg/mL of the composition according to an embodiment of the present invention.

FIG. 8 illustrates results obtained by evaluating inhibited expression level of inflammatory cytokines depending on doses of the composition of Example 7-1.

FIG. 9 illustrates results obtained by evaluating inhibited expression level of inflammatory cytokines depending on doses of the composition of Example 7-3.

FIG. 10 illustrates a plan for evaluating anti-atopic efficacy of the composition according to an embodiment of the present invention.

FIG. 11 illustrates results obtained by measuring body weight in evaluation of the anti-atopic efficacy of the composition according to an embodiment of the present invention.

FIG. 12 illustrates clinical symptom results (photographs) in evaluation of the anti-atopic efficacy of the composition according to an embodiment of the present invention.

FIG. 13 illustrates results (A) obtained by identifying skin irritation index and results (B) obtained by measuring ear thickness in evaluation of the anti-atopic efficacy of the composition according to an embodiment of the present invention.

FIG. 14 illustrates results obtained by measuring lymph node weight in evaluation of the anti-atopic efficacy of the composition according to an embodiment of the present invention.

**Best** Mode for Carrying out Invention

Hereinafter, the present invention will be described in more detail by way of specific examples. However, the present invention is not limited to the following examples, and it will be apparent to those skilled in the art that various modifications or variations can be made within the spirit and scope of the present invention. Accordingly, the appended claims should be construed broadly and in a manner consistent with the spirit and scope of the present invention.

**Example 1. Preparation of herbal medicinal extracts**

Each of Scutellaria baicalensis, Liriope platyphylla, Sophora flavescens, Dictamnus root bark, and Phellodendron bark was heated and stirred at a temperature of 50°C for 72 hours in a mixture of butylene glycol and water. Then, filtration was performed under reduced pressure through a filter paper. The filtered extract was refrigerated at a temperature of 4°C for 24 hours. Then, the extract was filtered again under reduced pressure to obtain an extract, which was refrigerated at a temperature of 4°C.

**Example 2. Preparation of composition containing extracts**

The extracts prepared in Example 1 were weighed as shown in Table 1 and mixed. The mixture was stirred, filtered again under reduced pressure through a filter paper, concentrated, and freeze-dried to prepare a composition containing the extracts. A comparative example, in which a composition ratio of the respective extracts was different, was used as a control.

[Table 1]

|  | **Scutellaria baicalensis** | Liriope platyphylla | **Sophora flavescens** | Dictamnus **root bark** | **Phellodendron bark** |
|---|---|---|---|---|---|
| Example 2 | 100 g | 100 g | 100 g | 100 g | 100 g |
| Comparative Example | 100 g | 100 g | 200 g | 100 g | 100 g |

**Example 3. Evaluation of antioxidant activity with DCFH-DA measurement**

To measure intracellular reactive oxygen species (ROS), a fluorescence assay using DCFH-DA (2',7'-dichlorofluorescein-diacetate) was performed. DCFH-DA is a non-polar molecule that can be easily introduced into cells. DCFH-DA is converted to fluorescent DCF in a case of being separated into DCFH, which is a non-fluorescent polar molecule, by an esterase, and then oxidized by intracellular reactive oxygen species. The DCFH-DA was measured with a fluorometer.

Specifically, human dermal fibroblasts (HDFn) were cultured in DMEM (supplemented with 10% FBS and containing 1% antibiotics) at a condition of 37°C and 5% $CO_2$. About $1 \times 10^5$ cells per well were dispensed into each well of a 96-well plate. Then, treatment with the composition of Example 2 at 0, 0.5, 1, and 2 v/v% was performed, and culture was performed.

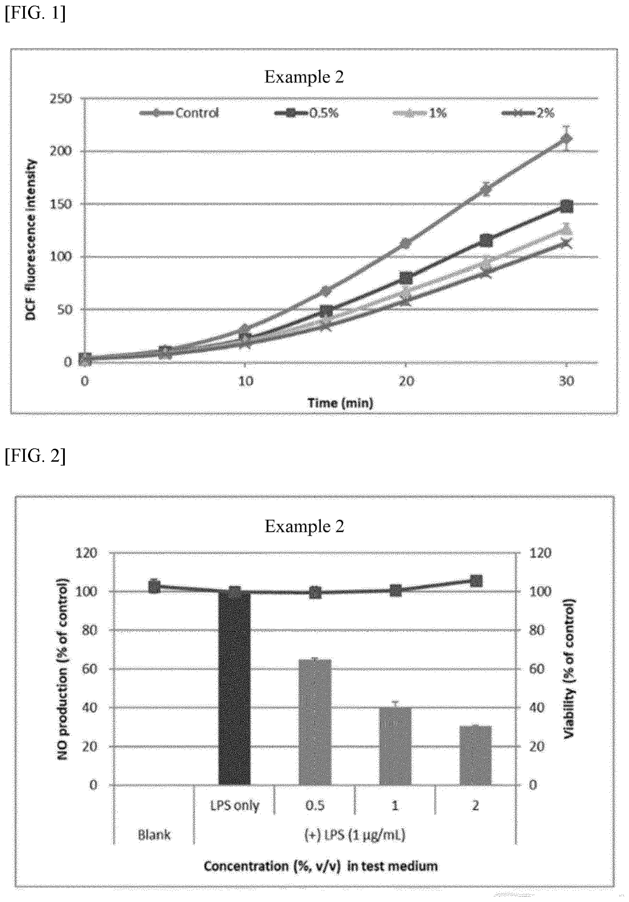

Then, 1 mM of $H_2O_2$, which is an oxidative stress-inducing substance, was added, and 20 μM of DCFH-DA (20 mM stock) was added. Then, the fluorescence reaction was analyzed with a spectrofluorometer (excitation 485 nm, emission 535 nm) at 37°C for 90 minutes, and the results are illustrated in FIG. 1. Treatment with the composition of Comparative Example at 2 v/v% was also respectively performed in the same manner. Then, the fluorescence reaction was analyzed, and the results are illustrated in Table 2. From each result, the reactive oxygen species inhibition ability (%) was quantified by calculating % of fluorescence intensity of the experimental group relative to the untreated control (0 v/v%).

[Table 2]

| Item | Reactive oxygen species inhibition ability (%) |
|---|---|
| Untreated control | 0% |
| Example 2-1 (2 v/v%) | 46.7% |
| Example 2-2 (3 v/v%) | 65.30% |
| Comparative Example (2 v/v%) | 40.90% |

As can be seen in FIG. 1, the antioxidant activity also increased as the concentration increased; and as can be seen in Table 2, it was identified that the highest ROS inhibitory effect was obtained at 3 v/v%. In particular, it was identified that the composition of Example 2 had an excellent effect as compared with the composition of Comparative Example, which contains twice the Sophora flavescens extract among the extracts, and thus had an excellent antioxidant effect.

**Example 4. Evaluation of NO synthesis inhibition ability**

NO, which is a pathological secondary signaling substance secreted by many cells, regulates activity of COX and acts synergistically during inflammatory responses. NO produced from NOS induced by LPS in Raw264.7 cells was measured to identify an inflammatory response inhibitory effect.

Raw264.7 cells were subjected to treatment with the composition of Example 2 at 0, 0.5, 1, and 2 v/v%, and then the NO synthesis inhibition ability was evaluated using a NO quantification kit based on Expression 1. The control was treated only with LPS without adding a sample. The results are illustrated in FIG. 2.

[Expression 1]

NO synthesis inhibition ability (%) = {1 - (Slope of NO synthesis amount when sample is added)}/(NO synthesis amount when no sample is added) × 100

As can be seen from Figure 2, the composition of Example 2 according to the present invention had an increased anti-inflammatory effect as the concentration increased. Referring to Table 3, it was found that the composition of Example 2 according to the present invention had the highest anti-inflammatory effect at 3 v/v%.

Treatment with the composition of Comparative Example at 2 v/v% was performed to analyze its anti-inflammatory effect in the same manner as above, and the results are shown in Table 3.

[Table 3]

| Item | NO synthesis inhibition ability (%) |
|---|---|
| Untreated control (LPS only) | 0% |
| Example 2-1 (2 v/v%) | 69.3% |
| Example 2-2 (3 v/v%) | 98% |
| Comparative Example | 65% |

From the above results, it was identified that the composition of Example 2 had an excellent effect as compared with the composition of Comparative Example, which contains twice the Sophora flavescens extract among the extracts, and thus had an excellent anti-inflammatory effect.

**Example 5. Evaluation of whitening efficacy through measurement of intracellular melanin production**

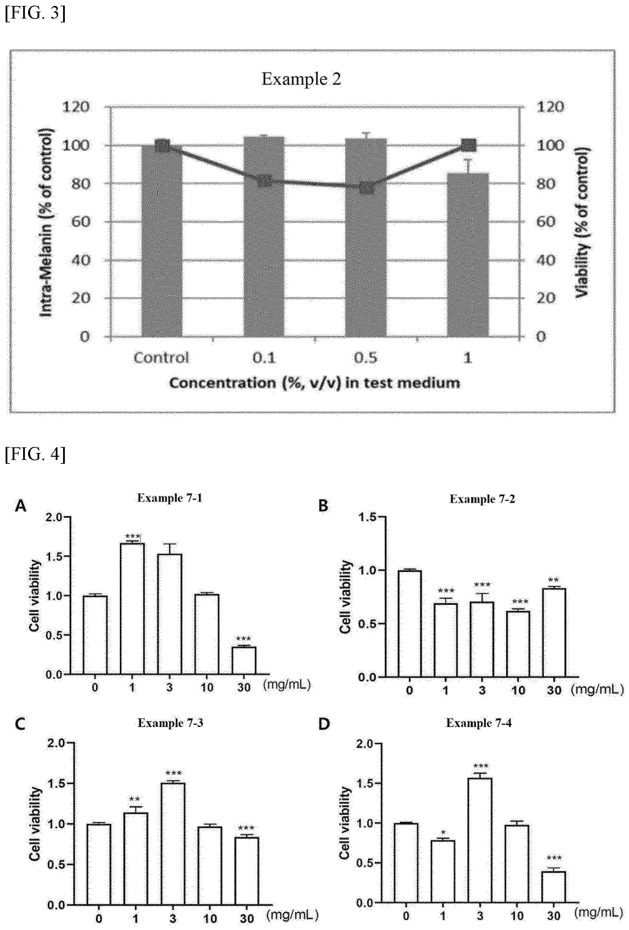

To identify a whitening effect of the extract, its effect on inhibition of melanin biosynthesis was measured using the mouse-derived melanocytes B16F1.
The B16F1 melanocytes were dispensed into each well and the cells were allowed to attach thereto. Then, treatment with the composition of Example 2 at 0, 0.5, 1, and 2 v/v and the composition of Comparative Example at 2 v/v% was performed, and incubation was performed. After incubation, the cells were detached with trypsin-EDTA. Subsequently, the number of cells was measured, and then the cells were collected by centrifugation. 1 N NaOH (10% DMSO) was added to the cell filtrate to extract melanin. The extracted melanin was dissolved, and the absorbance of melanin was measured with a microplate reader. Subsequently, melanin was quantified to measure a melanin production inhibition rate (%) of the sample. A melanin production inhibition rate (%) in the B16F1 melanocytes was calculated by Expression 2, and the results are shown in Table 4 and FIG. 3.

[Expression 2]

Melanin production inhibition rate (%) = {(Melanin amount when no sample is added) – (Melanin amount when sample is added)}/(Melanin amount when no sample is added) × 100

[Table 4]

| Item | Melanin production inhibition rate (%) |
|---|---|
| Untreated control (LPS only) | 0% |
| Example 2-1 (2 v/v%) | 14.5% |
| Comparative Example | 0% |

From the above results, it was identified that the composition of Example 2 inhibited melanin production in a concentration-dependent manner, and thus had whitening activity. In particular, from the viewpoint that the composition of Example 2 had a melanin production inhibition ability even though the composition of Comparative Example had no whitening effect, it was identified that the composition of the present invention had a specifically effective whitening effect.

**Example 6. Efficacy evaluation depending on extraction conditions**

To identify whether efficacy of the composition of the present invention varies depending on extraction methods, it was checked whether the respective compositions had antioxidant and anti-inflammatory effects in a case where extraction is performed using different extraction temperatures and times as shown in Table 5.

[Table 5]

| Item | Temperature | Time |
|---|---|---|
| Example 6-1 | 50°C | 72 hours |
| Example 6-2 | 60°C | 96 hours |
| Example 6-3 | 100°C | 1 and half hours |

For evaluation of antioxidant and anti-inflammatory effects, experiments were conducted in the same manner as in Examples 3 and 4. The sample at 2 v/v% was used.

[Table 6]

| Item | Reactive oxygen species inhibition ability (%) | NO synthesis inhibition ability (%) |
|---|---|---|
| Untreated control | 0% | 0% |
| Example 6-1 | 62% | 74.10% |
| Example 6-2 | 34.70% | 61.10% |
| Example 6-3 | 41.50% | 40.8.% |

The results are as shown in Table 6. It was identified that the active ingredients for antioxidant and anti-inflammatory properties were highly extracted under the extraction condition of 50°C for 72 hours, thereby allowing excellent efficacy.

**Example 7. Cytotoxicity evaluation (MTT assay)**

As a colorimetric assay to evaluate metabolic activity of cells, this experiment was performed. To evaluate toxicity of a test substance, viability of the cells treated with the test substance was compared with cell viability of a control which was regarded as 100%. It was determined that the test substance was toxic in a case where the cell viability was 70% or lower.

Specifically, cytotoxicity evaluation was performed in human-derived keratinocytes (HaCaT). The composition was prepared in the same manner as in Example 2, except that as shown in Table 7, distilled water 100% or aqueous solution of ethanol (30% ethanol) was used in place of butylene glycol and water to compare differences between the solvents, and spray drying was performed in addition to freeze drying to compare differences between the drying methods. Then, the cells were subjected to treatment with each of the prepared compositions of Example 7 at doses of 0, 1, 3, 10, and 30 mg/mL, respectively.

[Table 7]

| Item | Solvent | Drying method | Remarks |
|---|---|---|---|
| Example 7-1 | Distilled water 100% | Spray drying | Suspended matters are present |
| Example 7-2 | Distilled water 100% | Freeze drying | - |
| Example 7-3 | Distilled water 70% + Ethanol 30% | Spray drying | Suspended matters are present |
| Example 7-4 | Distilled water 70% + Ethanol 30% | Freeze drying | Suspended matters are present |

As a result, it was identified that Examples 7-1 to 7-4 were all non-cytotoxic at 10 mg/mL or lower (results of Example 7-1, Example 7-2, Example 7-3, and Example 7-4 are illustrated in FIG. 4A, FIG. 4B, FIG. 4C, and FIG. 4D, respectively).

**Example 8. Evaluation of NO production (NO assay)**

NO is a substance produced when cells are stimulated, and a concentration of NO released into culture medium was measured using a $NaNO_2$ standard curve. NO production of LPS (0.1 μg/mL), which is a representative inflammation-inducing substance, is around 30 μM, and LPS was used as a control. Specifically, treatment with the respective compositions of Examples 7-1 to 7-4 at 0, 0.1, 0.3, 1, and 3 mg/mL, respectively, was performed in the presence of LPS (0.1

μg/mL).

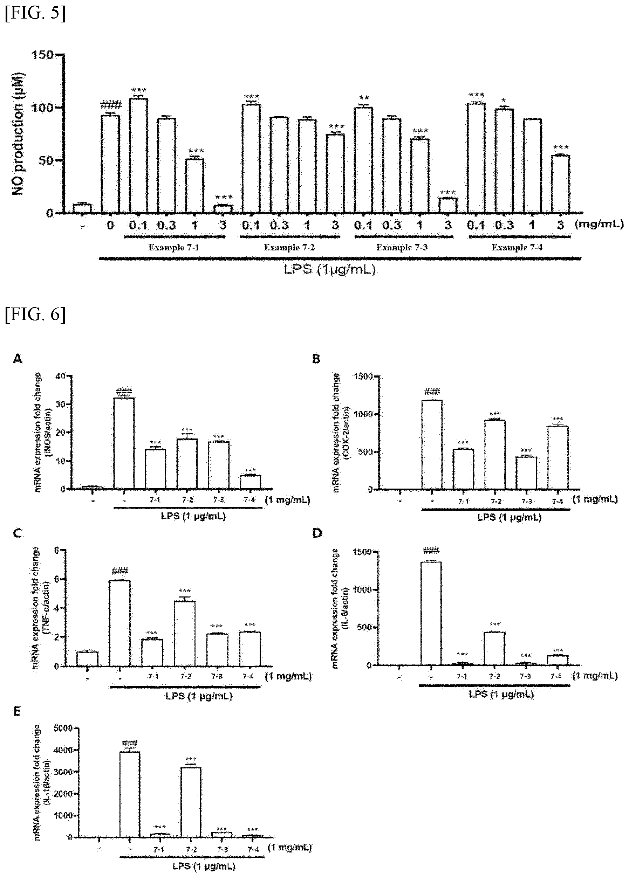

As a result, it was identified that the composition of Example 7-2 had no significant effect on a decrease in NO production; the compositions of Examples 7-3 and 7-4 decreased NO production at 3 mg/mL or higher; and the composition of Example 7-1 decreased NO production at 1 mg/mL or higher (FIG. 5).

**Example 9. Evaluation of expression level of inflammatory cytokines (RT-PCR)**

**(1) Evaluation of inhibited expression level of inflammatory cytokines caused by 1 mg/mL of compositions of Examples 7-1 to 7-4**

In a case where the cells are stimulated, the expression level of inflammatory cytokines (iNOS, COX-2, TNF-$\alpha$, IL-6, IL-1$\beta$) increases. Thus, an attempt was made to determine whether the expression of inflammatory cytokines was inhibited in a case where treatment with each of the compositions of Examples 7-1 to 7-4 at 1 mg/mL was performed. Specifically, intracellular mRNA was extracted and the expression level of inflammatory cytokines was measured through RT-PCR.

As a result, overall, the composition of Example 7-1 had the highest inhibitory effect on expression of inflammatory cytokines, followed by then the composition of Example 7-3, followed by the composition of Example 7-4, and followed by the composition of Example 7-2 (FIG. 6). Specifically, the two compositions having the highest inhibitory effect on expression of each inflammatory cytokine are shown in Table 8.

[Table 8]

| Item | iNOS | COX-2 | TNF-$\alpha$ | IL-6 | IL-1$\beta$ |
|---|---|---|---|---|---|
| Example 7-1 | O | O | O | O | O |
| Example 7-2 | - | - | - | - | - |
| Example 7-3 | - | O | O | O | - |
| Example 7-4 | O | - | - | - | O |

**(2) Evaluation of inhibited expression level of inflammatory cytokines caused by 3 mg/mL of compositions of Examples 7-1 to 7-4**

In addition, an attempt was made to determine whether the expression of inflammatory cytokines was inhibited in a case where treatment with each of the compositions of Examples 7-1 to 7-4 at 3 mg/mL was performed. Specifically, intracellular mRNA was extracted and the expression level of inflammatory cytokines was measured through RT-PCR.

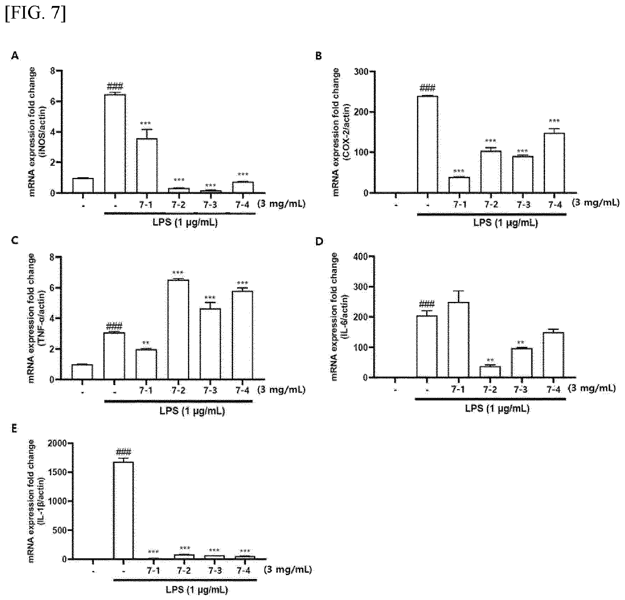

As a result, overall, the composition of Example 7-1 had the highest inhibitory effect on expression of inflammatory cytokines, followed by then the composition of Example 7-3, followed by the composition of Example 7-2, and followed by the composition of Example 7-4 (FIG. 7). Specifically, the two compositions having the highest inhibitory effect on expression of each inflammatory cytokine are shown in Table 9.

[Table 9]

| Item | iNOS | COX-2 | TNF-$\alpha$ | IL-6 | IL-1$\beta$ |
|---|---|---|---|---|---|
| Example 7-1 | - | O | O | - | O |
| Example 7-2 | O | - | - | O | - |
| Example 7-3 | O | O | - | O | - |
| Example 7-4 | - | - | - | - | O |

**(3) Evaluation of inhibited expression level of inflammatory cytokines depending on doses of composition of Example 7-1**

The composition of Example 7-1 had the highest inhibitory effect on expression of inflammatory cytokines. Thus, the expression level of inflammatory cytokines depending on doses of the composition of Example 7-1 was evaluated.

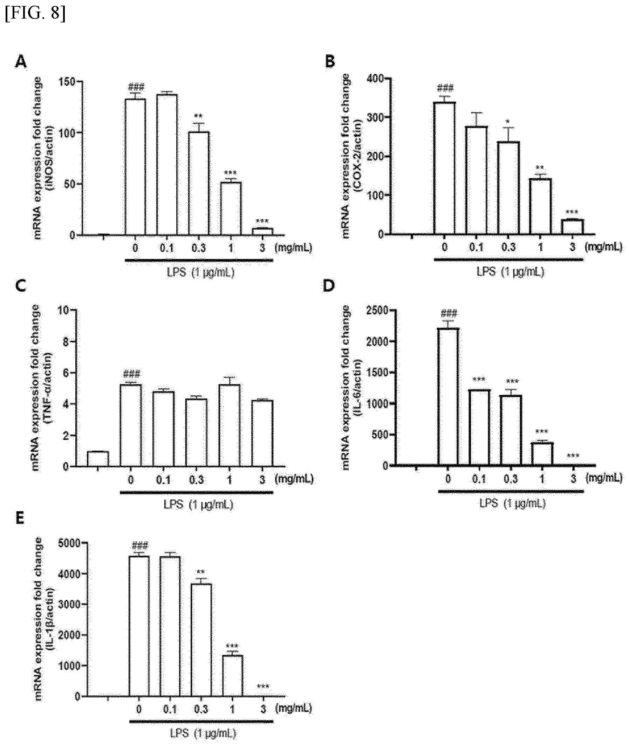

As a result, it was identified that the composition had a better inhibitory effect on expression of inflammatory cytokines (except TNF-α) as the dose increased; and most of the inflammatory cytokines (except TNF-α) were inhibited in a case where treatment with the composition at 3 mg/mL was performed (FIG. 8).

**(4) Evaluation of inhibited expression level of inflammatory cytokines depending on doses of composition of Example 7-3**

The composition of Example 7-3 had the second-highest inhibitory effect on expression of inflammatory cytokines following the composition of Example 7-1. Thus, the expression level of inflammatory cytokines depending on doses of the composition of Example 7-3 was evaluated.

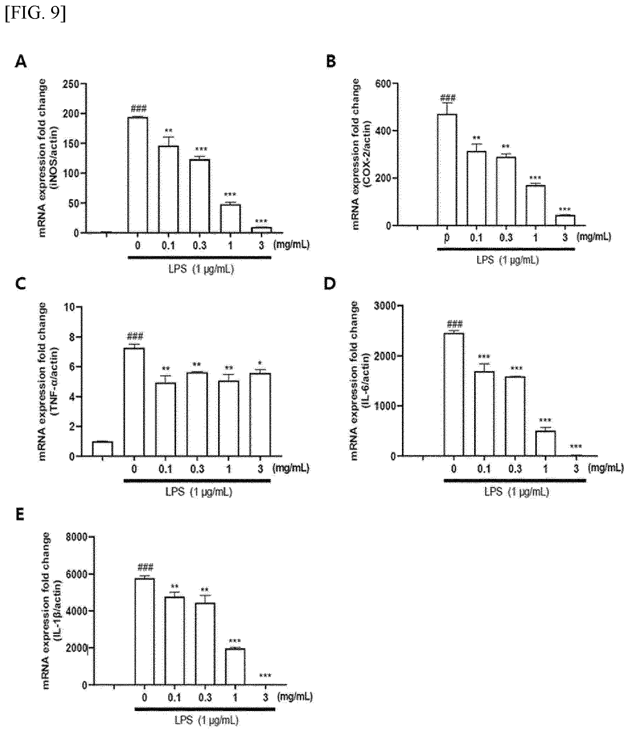

As a result, it was identified that the composition had a better inhibitory effect on expression of inflammatory cytokines (except TNF-α) as the dose increased; and most of the inflammatory cytokines (except TNF-α) were inhibited in a case where treatment with the composition at 3 mg/mL was performed (FIG. 9).

**Example 10. Evaluation of anti-atopic efficacy**

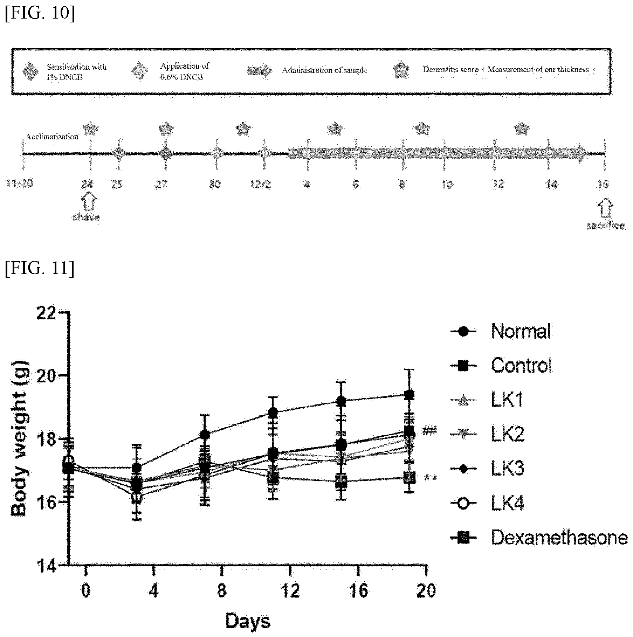

To evaluate *in vivo* anti-atopic efficacy, mice were prepared. The mice were acclimatized for 4 days, and then the hairs of the mice were shaved. One day after the hair removal, the mice were sensitized with 1% 2,4-dinitrochlorobenzene (DNCB). Two days after DNCB sensitization, the mice were sensitized once more with 1% DNCB. Starting from 3 days after the second DNCB sensitization, 0.6% DNCB was applied to the hair-removed area at two-day intervals for a total of 8 times. The next day after the second application of DNCB, the mice were orally administered with 500 mpk each of the compositions of Examples 7-1 to 7-4, and 1 mpk of dexamethasone and 1 mpk of 0.9% saline as controls, to evaluate their efficacy. To evaluate the results, evaluation of skin irritation (dermatitis score), measurement of ear thickness, measurement of body weight, and evaluation of clinical symptoms (photographs) were performed over a total of 6 times on the day of hair removal, the day of second DNCB sensitization, the day after the first DNCB application, and at intervals of 4 days starting from the second day after administration of each composition. The mice were sacrificed 13 days after administration of each composition. Subsequently, the lymph nodes of the sacrificed mice were weighed (Table 10 and FIG. 10).

[Table 10]

| Test groups | Induction of atopy | Substance administered | Route of administration | Number of animals |
|---|---|---|---|---|
| Normal | - | 0.9% saline 1 mpk | Oral | 8 |
| Control | Snsitization with 1% DNCB + Application of 6% DNCB | 0.9% saline 1 mpk | | 8 |
| Dexamethasone | | Dexamethasone 1 mpk | | 8 |
| LK1 | | Example 7-1 500 mpk | | 8 |
| LK2 | | Example 7-2 500 mpk | | 8 |
| LK3 | | Example 7-3 500 mpk | | 8 |
| LK4 | | Example 7-4 500 mpk | | 8 |

From the results of body weight measurement, it was found that the groups administered with the compositions of Examples 7-1 to 7-4 gained less weight than the control group (FIG. 11).

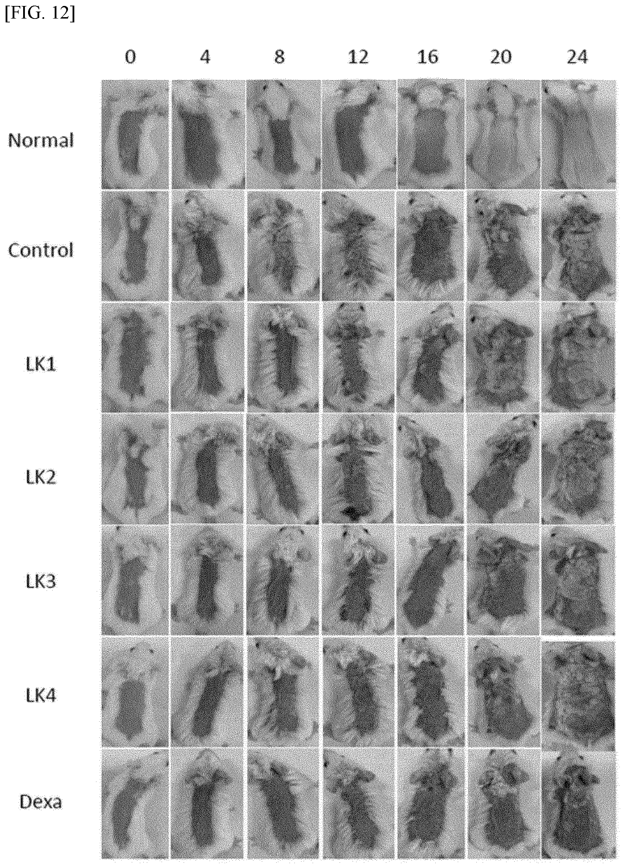

In addition, the clinical symptoms were directly checked by observing the affected area. As a result, it was visually found that for all groups administered with the compositions of Examples 7-1 to 7-4, the affected area was improved as compared with the control group; and in particular, it was found that for the group administered with the composition of Examples 7-3, the affected area was improved as compared with the control group (FIG. 12).

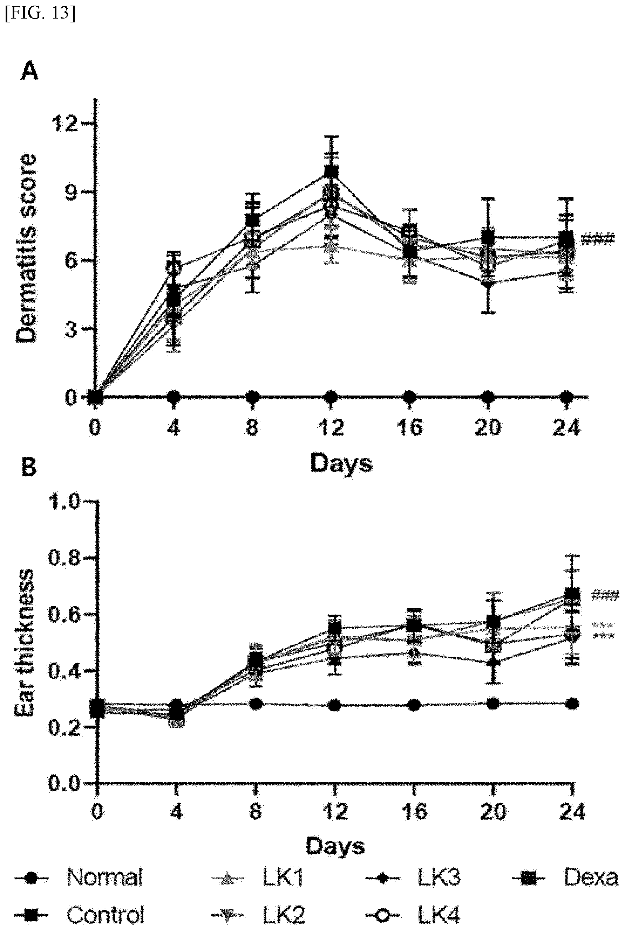

In addition, after oral administration for 2 weeks, the symptoms of atopic dermatitis were evaluated by skin irritation index. As a result, it was found that all groups administered with the compositions of Examples 7-1 to 7-4 had a lower index than the control group; and in particular, it was found that the composition of Example 7-3 had a lower skin irritation index than dexamethasone (FIG. 13A).

In addition, from the results of ear thickness measurement, it was found that for the groups administered with the compositions of Examples 7-1, 7-3, and 7-4, the ear thickness was significantly decreased (FIG. 13B).

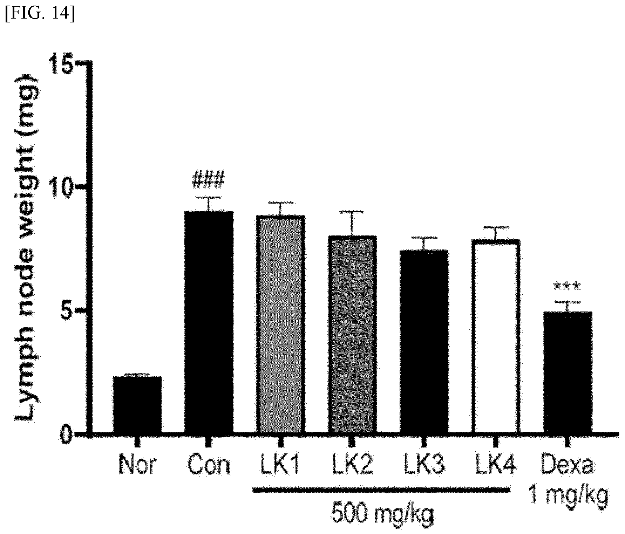

In addition, the lymph node weight was measured after sacrifice of each mouse. As a result, it was found that for all groups administered with the compositions of Examples 7-1 to 7-4, the lymph node weight was decreased as compared with the control group (FIG. 14).

Overall, it was found that the composition of Example 7-3 exhibited the best effects.

## Claims

1. A pharmaceutical composition for preventing or treating a skin-related disease, comprising at least one extract selected from the group consisting of a Scutellaria baicalensis extract, a Liriope platyphylla extract, a Sophora flavescens extract, a Dictamnus root bark extract, and a Phellodendron bark extract.

2. The pharmaceutical composition of claim 1, wherein the composition comprises the Scutellaria baicalensis extract, the Liriope platyphylla extract, the Sophora flavescens extract, the Dictamnus root bark extract, and the Phellodendron bark extract.

3. The pharmaceutical composition of claim 2, wherein the composition comprises 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, based on 100 parts by weight of the Scutellaria baicalensis extract.

4. The pharmaceutical composition of claim 1, wherein a content of the extract in the composition is 0.5 v/v% to 5 v/v%.

5. The pharmaceutical composition of claim 1, wherein the composition is administered at a concentration of 0.1 to 10 mg/mL.

6. The pharmaceutical composition of claim 1, wherein the composition is administered at a dose of 100 to 1000 mpk.

7. The pharmaceutical composition of claim 1, wherein the extract is obtained by extraction with at least one solvent selected from the group consisting of water, an organic solvent, and a mixture thereof.

8. The pharmaceutical composition of claim 1, wherein the skin-related disease is at least one disease selected from the group consisting of skin wrinkles, photoaging, melanosis, skin pigmentation, skin burns, skin inflammation, skin cancer, melasma, freckles, lentigines, nevus, psoriasis, atopic dermatitis, alopecia areata, vitiligo, athlete's foot, and dandruff.

9. A method for preventing or treating a skin-related disease, comprising a step of administering the pharmaceutical composition of claim 1 to an individual in need thereof.

10. A preparation method of a pharmaceutical composition for the prevention or treatment of a skin-related disease, comprising a step of extracting at least one natural material selected from the group consisting of Scutellaria baicalensis, Liriope platyphylla, Sophora flavescens, Dictamnus root bark, and Phellodendron bark.

11. The preparation method of claim 10, wherein the natural material includes all of the Scutellaria baicalensis, the Liriope platyphylla, the Sophora flavescens, the Dictamnus root bark, and the Phellodendron bark, provided that the extraction is carried out for each of the Scutellaria baicalensis, the Liriope platyphylla, the Sophora flavescens, the Dictamnus root bark, and the Phellodendron bark.

12. The preparation method of claim 11, further comprising a step of mixing, based on 100 parts by weight of the Scutellaria baicalensis extract, 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, to obtain a mixture.

13. The preparation method of claim 12, further comprising a step of drying the mixture.

**14.** The preparation method of claim 13, wherein the drying is spray drying.

**15.** The preparation method of claim 10, wherein the extraction is carried out at a temperature of 40 to 55°C.

**16.** The preparation method of claim 10, wherein the extraction is carried out for 60 to 80 hours.

**17.** The preparation method of claim 10, wherein the extraction is carried out with at least one solvent selected from the group consisting of water, an organic solvent, and a mixture thereof.

**18.** A pharmaceutical composition for preventing or treating a skin-related disease in animals, comprising at least one extract selected from the group consisting of a Scutellaria baicalensis extract, a Liriope platyphylla extract, a Sophora flavescens extract, a Dictamnus root bark extract, and a Phellodendron bark extract.

**19.** The pharmaceutical composition of claim 18, wherein the composition comprises the Scutellaria baicalensis extract, the Liriope platyphylla extract, the Sophora flavescens extract, the Dictamnus root bark extract, and the Phellodendron bark extract.

**20.** The pharmaceutical composition of claim 19, wherein the composition comprises 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, based on 100 parts by weight of the Scutellaria baicalensis extract.

**21.** The pharmaceutical composition of claim 18, wherein a content of the extract in the composition is 0.5 v/v% to 5 v/v%.

**22.** The pharmaceutical composition of claim 18, wherein the composition is administered at a concentration of 0.1 to 10 mg/mL.

**23.** The pharmaceutical composition of claim 18, wherein the composition is administered at a dose of 100 to 1000 mpk.

**24.** The pharmaceutical composition of claim 18, wherein the extract is obtained by extraction with at least one solvent selected from the group consisting of water, an organic solvent, and a mixture thereof.

**25.** The pharmaceutical composition of claim 18, wherein the skin-related disease is at least one disease selected from the group consisting of skin wrinkles, photoaging, melanosis, skin pigmentation, skin burns, skin inflammation, skin cancer, melasma, freckles, lentigines, nevus, psoriasis, atopic dermatitis, alopecia areata, vitiligo, athlete's foot, and dandruff.

**26.** The pharmaceutical composition of claim 18, wherein the animal is a mammal.

**27.** A method for preventing or treating a skin-related disease in animals, comprising a step of administering the pharmaceutical composition of claim 18 to an individual in need thereof.

**28.** A preparation method of a pharmaceutical composition for the prevention or treatment of a skin-related disease in animals, comprising a step of extracting at least one natural material selected from the group consisting of Scutellaria baicalensis, Liriope platyphylla, Sophora flavescens, Dictamnus root bark, and Phellodendron bark.

**29.** The preparation method of claim 28, wherein the natural material includes all of the Scutellaria baicalensis, the Liriope platyphylla, the Sophora flavescens, the Dictamnus root bark, and the Phellodendron bark, provided that the extraction is carried out for each of the Scutellaria baicalensis, the Liriope platyphylla, the Sophora flavescens, the Dictamnus root bark, and the Phellodendron bark.

**30.** The preparation method of claim 29, further comprising a step of mixing, based on 100 parts by weight of the Scutellaria baicalensis extract, 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, to obtain a mixture.

**31.** The preparation method of claim 30, further comprising a step of drying the mixture.

32. The preparation method of claim 31, wherein the drying is spray drying.

33. The preparation method of claim 28, wherein the extraction is carried out at a temperature of 40 to 55°C.

34. The preparation method of claim 28, wherein the extraction is carried out for 60 to 80 hours.

35. The preparation method of claim 28, wherein the extraction is carried out with at least one solvent selected from the group consisting of water, an organic solvent, and a mixture thereof.

36. A food composition for preventing or ameliorating a skin-related disease, comprising at least one extract selected from the group consisting of a Scutellaria baicalensis extract, a Liriope platyphylla extract, a Sophora flavescens extract, a Dictamnus root bark extract, and a Phellodendron bark extract.

37. The food composition of claim 36, wherein the composition comprises the Scutellaria baicalensis extract, the Liriope platyphylla extract, the Sophora flavescens extract, the Dictamnus root bark extract, and the Phellodendron bark extract.

38. The food composition of claim 37, wherein the composition comprises 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, based on 100 parts by weight of the Scutellaria baicalensis extract.

39. The food composition of claim 36, wherein a content of the extract in the composition is 0.5 v/v% to 5 v/v%.

40. The food composition of claim 36, wherein the composition is administered at a concentration of 0.1 to 10 mg/mL.

41. The food composition of claim 36, wherein the composition is administered at a dose of 100 to 1000 mpk.

42. The food composition of claim 36, wherein the extract is obtained by extraction with at least one solvent selected from the group consisting of water, an organic solvent, and a mixture thereof.

43. The food composition of claim 36, wherein the skin-related disease is at least one disease selected from the group consisting of skin wrinkles, photoaging, melanosis, skin pigmentation, skin burns, skin inflammation, skin cancer, melasma, freckles, lentigines, nevus, psoriasis, atopic dermatitis, alopecia areata, vitiligo, athlete's foot, and dandruff.

44. A method for preventing or ameliorating a skin-related disease, comprising a step of causing an individual in need thereof to ingest the food composition of claim 36.

45. A preparation method of a food composition for the prevention or amelioration of a skin-related disease, comprising a step of extracting at least one natural material selected from the group consisting of Scutellaria baicalensis, Liriope platyphylla, Sophora flavescens, Dictamnus root bark, and Phellodendron bark.

46. The preparation method of claim 45, wherein the natural material includes all of the Scutellaria baicalensis, the Liriope platyphylla, the Sophora flavescens, the Dictamnus root bark, and the Phellodendron bark, provided that the extraction is carried out for each of the Scutellaria baicalensis, the Liriope platyphylla, the Sophora flavescens, the Dictamnus root bark, and the Phellodendron bark.

47. The preparation method of claim 46, further comprising a step of mixing, based on 100 parts by weight of the Scutellaria baicalensis extract, 50 to 150 parts by weight of the Liriope platyphylla extract, 50 to 150 parts by weight of the Sophora flavescens extract, 50 to 150 parts by weight of the Dictamnus root bark extract, and 50 to 150 parts by weight of the Phellodendron bark extract, to obtain a mixture.

48. The preparation method of claim 47, further comprising a step of drying the mixture.

49. The preparation method of claim 48, wherein the drying is spray drying.

50. The preparation method of claim 45, wherein the extraction is carried out at a temperature of 40 to 55°C.

51. The preparation method of claim 45, wherein the extraction is carried out for 60 to 80 hours.

52. The preparation method of claim 45, wherein the extraction is carried out with at least one solvent selected from the group consisting of water, an organic solvent, and a mixture thereof.

[FIG. 1]

[FIG. 2]

[FIG. 3]

Example 2

[FIG. 4]

A — Example 7-1

B — Example 7-2

C — Example 7-3

D — Example 7-4

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

<div align="center">

**INTERNATIONAL SEARCH REPORT**

</div>

| International application No. |
|---|
| **PCT/KR2021/000986** |

**A. CLASSIFICATION OF SUBJECT MATTER**

**A61K 36/53**(2006.01)i; **A61K 36/896**(2006.01)i; **A61K 36/489**(2006.01)i; **A61K 36/75**(2006.01)i; **A61K 36/756**(2006.01)i; **A61P 17/00**(2006.01)i; **A61P 39/06**(2006.01)i; **A61P 29/00**(2006.01)i

According to International Patent Classification (IPC) or to both national classification and IPC

**B. FIELDS SEARCHED**

Minimum documentation searched (classification system followed by classification symbols)

A61K 36/53(2006.01); A61K 36/232(2006.01); A61K 36/482(2006.01); A61K 36/708(2006.01); A61K 8/97(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 황금(scutellaria baicalensis), 맥문동(broadleaf liriope), 고삼(sophora flavescens), 백선피(dictamnus dasycarpus), 황백(rutaceae), 추출(extraction), 피부 관련 질환(skin related disease), 제약(drug), 식품(food)

**C. DOCUMENTS CONSIDERED TO BE RELEVANT**

| Category* | Citation of document, with indication, where appropriate, of the relevant passages | Relevant to claim No. |
|---|---|---|
| X | KR 10-1799516 B1 (LEE, Jong Ryul et al.) 20 November 2017 (2017-11-20)<br>See paragraphs [0016], [0021]-[0024], [0028], [0035], [0039], [0041], [0046]-[0050] and [0055]-[0065], example 5, and tables 1-3. | 1-8,10-26,28-43,45-52 |
| A | KR 10-2015-0088154 A (LIOELE COSMETIC CO., LTD.) 31 July 2015 (2015-07-31)<br>See entire document. | 1-8,10-26,28-43,45-52 |
| A | KR 10-2018-0025689 A (UNIVERSITY-INDUSTRY COOPERATION GROUP OF KYUNG HEE UNIVERSITY) 09 March 2018 (2018-03-09)<br>See entire document. | 1-8,10-26,28-43,45-52 |
| A | KR 10-2003-0055154 A (HANIRO, INC.) 02 July 2003 (2003-07-02)<br>See entire document. | 1-8,10-26,28-43,45-52 |
| A | KR 10-2006-0118809 A (KIM, Jeong Jin) 24 November 2006 (2006-11-24)<br>See entire document. | 1-8,10-26,28-43,45-52 |

☐ Further documents are listed in the continuation of Box C.　　☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**30 April 2021** | Date of mailing of the international search report<br><br>**30 April 2021** |
| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2021/000986** |

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☑ Claims Nos.: **9, 27, 44**
   because they relate to subject matter not required to be searched by this Authority, namely:

   Claims 9, 27 and 44 pertain to a method for treatment of the human body by therapy (PCT Article 17(2)(a) (i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2019)

INTERNATIONAL SEARCH REPORT
Information on patent family members

| Patent document cited in search report | | | Publication date (day/month/year) | Patent family member(s) | | | Publication date (day/month/year) |
|---|---|---|---|---|---|---|---|
| KR | 10-1799516 | B1 | 20 November 2017 | WO | 2018-203668 | A1 | 08 November 2018 |
| KR | 10-2015-0088154 | A | 31 July 2015 | KR | 10-1760418 | B1 | 21 July 2017 |
| KR | 10-2018-0025689 | A | 09 March 2018 | KR | 10-1842786 | B1 | 27 March 2018 |
| KR | 10-2003-0055154 | A | 02 July 2003 | | None | | |
| KR | 10-2006-0118809 | A | 24 November 2006 | KR | 10-0742378 | B1 | 24 July 2007 |
| | | | | WO | 2006-123887 | A1 | 23 November 2006 |

International application No.

**PCT/KR2021/000986**

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **N. SIVARANJANI.** *Journal of Clinical and Diagnostic Research,* 2013, vol. 7 (12), 2683-2685